Europäisches Patentamt

European Patent Office

Office européen des brevets

(11) Numéro de publication: **0 406 122 A1**

# (12) DEMANDE DE BREVET EUROPEEN

(21) Numéro de dépôt: 90401891.8

(22) Date de dépôt: 29.06.90

(51) Int. Cl.5: **C12N 15/12, C12P 21/02**

(30) Priorité: 30.06.89 FR 8908852

(43) Date de publication de la demande:
02.01.91 Bulletin 91/01

(84) Etats contractants désignés:
AT BE CH DE DK ES FR GB GR IT LI LU NL SE

(71) Demandeur: **ASSOCIATION POUR LE DEVELOPPEMENT DE LA RECHERCHE EN GENETIQUE MOLUCULAIRE, (A.DE.RE.GE.M)**
**23 rue Montpensier**
**F-75001 Paris(FR)**

(72) Inventeur: **Metzger, Daniel**
**37 rue Fin-de-Banlieue**
**F-67400 Illkirch-Graffenstaden(FR)**
Inventeur: **Tora, Lazlo**
**2 rue St Marc**
**F-67000 Strasbourg(FR)**
Inventeur: **Chambon, Pierre**
**200 rue du Docteur Schweitzer**
**F-67113 Blaesheim(FR)**

(74) Mandataire: **Warcoin, Jacques et al**
**Cabinet Régimbeau 26, avenue Kléber**
**F-75116 Paris(FR)**

(54) Procédé d'obtention d'un récepteur humain natif sauvage des oestrogènes et ses applications.

(57) La présente concerne un procédé d'obtention du récepteur humain natif sauvage des oestrogènes, caractérisé en ce qu'on cultive des cellules recombinantes incorporant l'ADN codant pour les récepteurs humains natifs sauvages des oestrogènes comportant une glycine en position 400 sous le contrôle d'élément assurant l'expression de cet ADN dans les cellules en cause, et en ce que l'on purifie ledit récepteur si nécessaire.

EP 0 406 122 A1

## PROCEDE D'OBTENTION D'UN RECEPTEUR HUMAIN NATIF SAUVAGE DES OESTROGENES ET SES APPLICATIONS

La présente invention concerne l'expression du récepteur humain natif "sauvage" des oestrogènes et l'application de ce récepteur notamment à titre de modèle utilisable en pharmacologie et son diagnostic.

Le document WO 87/05049 décrit l'expression dans les cellules eucaryotes de protéines récepteur des stéroïdes et notamment un récepteur des oestrogènes.

Le récepteur décrit dans ce brevet est un récepteur artificiel des oestrogènes dont les caractéristiques sont modifiées par la température. Ainsi, on a maintenant pu mettre en évidence qu'une température de 25°C affecte la capacité de ce récepteur à se lier à l'oestradiol, ce qui tient probablement à une instabilité du récepteur à cette température, en l'absence d'oestradiol. Cet inconvénient est particulièrement gênant dans le cas où l'on souhaite utiliser ledit récepteur dans des conditions physiologiques, c'est-à-dire à 37°C.

C'est pourquoi, la présente invention concerne le récepteur humain natif "sauvage" des oestrogènes qui permet notamment d'effectuer des essais de fixation dans des conditions physiologiques à la différence des récepteurs décrits dans le document WO 87/05049.

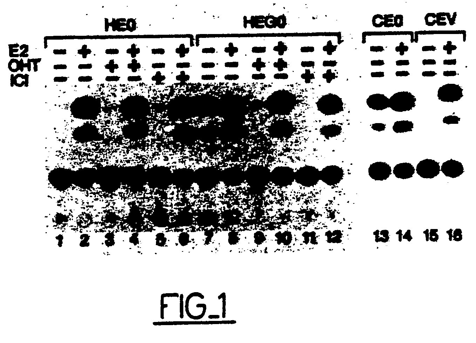

Le récepteur selon l'invention présente par rapport au récepteur décrit dans la technique antérieure, notamment dans la demande de brevet précédente, une glycine en position 400 au lieu d'une valine (voir figures 1-2 de la demande de brevet en cause). Ce récepteur natif sauvage comportant une glycine sera parfois dénommé HEGO et le récepteur de la technique antérieure comportant une valine HEO.

L'intérêt de ce type de récepteur a été largement décrit dans le brevet mentionné.

Tout d'abord, la production de quantité importante de récepteurs sauvages permet d'étudier sa structure fine et par là même de prévoir les structures possibles pour de nouveaux agonistes ou antagonistes des oestrogènes.

Ces nouveaux produits sont très importants dans la thérapie des affections à médiation stéroïdienne.

Le récepteur en lui-même sous forme purifiée ou exprimée dans des cellules est également utilisable pour étudier in vitro ou in vivo les activités de produits agonistes ou antagonistes, il s'agit là d'un modèle pharmacologique particulièrement intéressant car à la différence des récepteurs décrits précédemment, il peut être testé même à 25°C et également dans les conditions physiologiques, ce qui permet de mieux rendre compte des conditions réelles d'utilisation.

Enfin, ce récepteur permet de standardiser et de calibrer les immunoessais qui sont par exemple utilisés pour le diagnostic et le pronostic de certaines tumeurs, en particulier les cancers du sein.

L'invention concerne plus particulièrement un procédé d'obtention du récepteur humain natif sauvage des oestrogènes, caractérisé en ce qu'on cultive des cellules recombinantes incorporant l'ADN codant pour le récepteur humain natif sauvage des oestrogènes comportant une glycine en position 400 sous le contrôle d'éléments assurant l'expression de cet ADN dans les cellules en cause et en ce que l'on purifie ledit récepteur si nécessaire.

On utilisera la terminologie "récepteur humain natif sauvage des oestrogènes" aussi bien pour désigner le récepteur complet que le domaine isolé de liaisons des oestrogènes.

Parmi les cellules utilisables comme cellules hôtes, il faut citer en particulier les cellules animales (en particulier les cellules HeLa) et les cellules de levures.

Les élément assurants l'expression de la séquence d'ADN dans de telles cellules sont connus et figurent notamment dans la demande de brevet WO 87/05049 et également dans les documents en référence qui sont cités dans les exemples.

De préférence, dans la séquence codant pour le récepteur sauvage, la glycine en position 400 sera codée par le triplé GGG.

D'autres caractéristiques et avantages de la présente invention apparaîtront à la lecture des exemples suivants qui se réfèrent aux dessins ci-annexés :

**Figure 1 : Effet de la mutation remplaçant la glycine-400 par une valine dont le récepteur aux oestrogènes (Er) humain et du poulet sur l'activation de la transcription**

On a transfecté des cellules Hela avec 50 ng de vecteurs d'expression de Er humain ou de Er de poulet comme indiqué, conjointement avec 1 $\mu$g de plasmide "reporter" vit-tk-cat, et le plasmide pCH 110 de contrôle interne de l'expression de la béta-galactosidase (3$\mu$g) et de l'ADN transporteur (Bluescribe M13 +) pour un total de 20 $\mu$g d'ADN. On a maintenu les cultures pendant 48 heures en absence (-) ou en présence (+) de $10^{-8}$ M d'oestradiol (E2), puis on a dosé l'activité de l'enzyme CAT après normalisation pour l'activité de la béta-galactosidase (Wesbter et al., 1988). Des anti-hor-

2

mones ont été ajoutées comme indiqué en utilisant les concentrations suivantes : 4-hydroxy-tamoxifen (OHT), $10^{-8}$ M ; ICI 164,384 (ICI), $10^{-7}$ M.

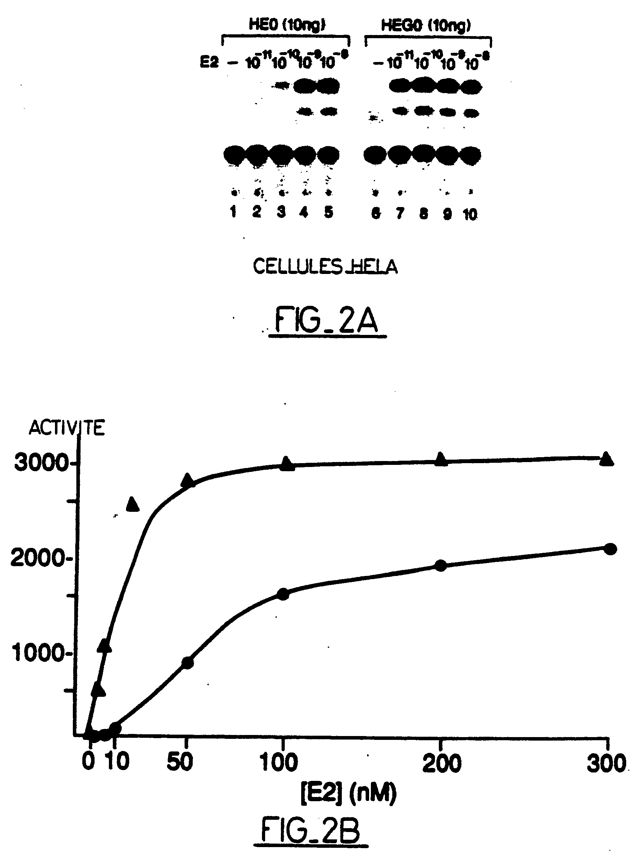

## Figure 2 : Effet de concentrations croissantes d'oestradiol sur l'activation de la transcription par le type sauvage (Gly-400) et mutant (val-400) de hER

A) Des cellules HeLa ont été transfectées avec 10 ng des vecteurs d'expression de l'hER correspondant, HEO (colonnes 1-5), ou HEGO (colonnes 6-10), conjointement avec 1 $\mu$G de plasmide "reporter" vit-tk-CAT, 3 $\mu$G du plasmide de contrôle de la béta-galactosidase pCH110 et avec de l'ADN Bluescribe M13+ pour un total d'ADN de 20 $\mu$g. Les cellules ont été maintenues pendant 48 heures en absence (-) ou en présence de différentes concentrations en oestradiol (E2) comme indiqué et dosées pour l'activité de l'enzyme CAT.

B) Des cellules de levures ont été transformées avec pYERE1/HER (cercles noirs) exprimant le mutant hER Val-400, et avec pYERE1/HEGO (triangles noirs) exprimant le type sauvage hER Gly-400. Ces vecteurs expriment aussi le gène d'Escherichia coli LacZ sous le contrôle d'un promoteur qui contient un ERE situé en amont de la séquence TATA du gène GAL1 (Metzger et al., 1988). On a cultivé les cellules de levures comme décrit (Metzger et al., 1988) et à $OD_{600}$ = 0,6 de l'oestradiol (E2) a été ajouté comme indiqué. 2 heures plus tard on a récolté les cellules de levures et un dosage de la béta-galactosidase a été effectué.

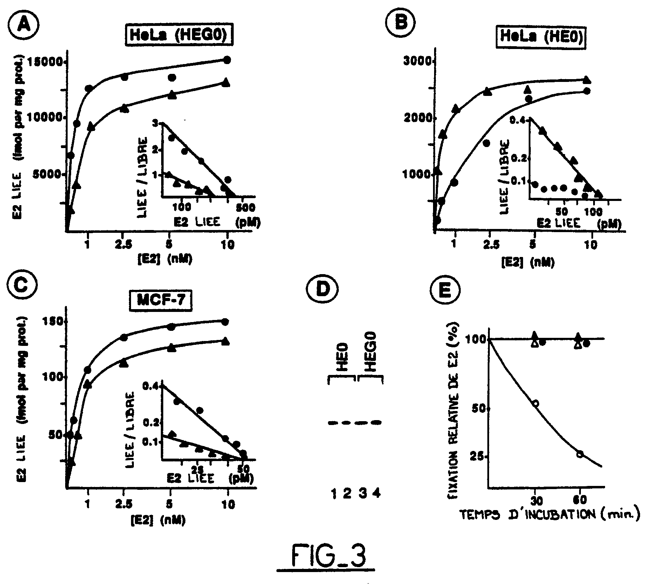

## Figure 3 : L'affinité apparente pour l'oestradiol du hER codé par HEO est altérée à 25°C

Les exraits cytosoliques préparés à partir de cellules HeLa transfectées avec soit 10 $\mu$g de HEGO (A) ou 10 $\mu$g de HEO (B), ou à partir de cellules MCF-7 (C) ont été soit incubés pendant 2 heures à 25°C (cercles noirs) ou toute une nuit à 0°C (triangles noirs) en présence de [3H]-17béta-oestradiol (E2) avec ou sans un excès d'un facteur 200 de 17béta-oestradiol non marqué. On a enlevé les stéroïdes non fixés en utilisant du charbon enrobé de dextrane. La fixation stéroïdienne a été mesurée par comptage de scintillation et la fixation spécifique a été calculée après soustraction de la fixation non spécifique (Kumar et al., 1986). Les figures incluses montrent les représentations de Scatchard de ces données. (D) Analyse au Western blot des extraits cytosoliques avant (colonne 1, HEO et colonne 3, HEGO) et après (colonne 2, HEO et colonne 4, HEGO), incubation à 25°C pendant 2 heures, en utilisant les anticorps monoclonaux H222 et D75 (Greene et al., 1984). (E) Des extraits cytosoliques préparés à partir de cellules HeLa transfectées avec 10 $\mu$g de HEGO ou de HEO ont été incubés à 25°C, soit sans oestrogène (triangles blancs pour HEGO, cercles blancs pour HEO), ou en présence de 10 nM de [3H]- 17 béta-oestradiol (triangles noirs pour HEGO, cercles noirs pour HEO). Après une incubation de 30 ou 60 minutes, on a ajouté 10 nM de [3H]-17 béta-oestradiol aux échantillons sans hormone et tous les échantillons ont été incubés pendant 2 heures de plus à 25°C. La fixation non spécifique a été déterminée dans des incubations parallèles en présence d'un excès d'un facteur de 200 de 17 béta-oestradiol non marqué. Les stéroïdes liés au récepteur ont été déterminés comme ci-dessus. 100 % de fixation oestrogène correspond à des extraits incubés avec de l'oestradiol marqué pendant 2 heures.

## Figure 4 :

(A) Représentation schématique de l'analyse par amplification PCR et par cartographie à la RNase pour détecter les substitutions simple-base dans l'ARNm d'hER et dans des échantillons d'ADN génomiques de cellules ER-positives et négatives.

De l'ADNc simple-brin a été synthétisé en utilisant l'ARN cellulaire total, la transcriptase réverse (RTase) et l'oligonucléotide "a" (Matériels et Méthodes). Les séquences correspondant à l'exon 5 long de 140 bp de hER ont été "PCR-amplifiées" à partir d'ADNc et d'ADN génomique, en utilisant comme amorce les oligonucléotides "a" et "b" (Matériels et Méthodes). On a synthétisé des sondes anti-sens (AS) HEGO et HEO en utilisant la polymérase T7 (pol) et des plasmides Bluescribe M13+ linéarisés (BSM) contenant les séquences d'ADNc d'hER. Les sondes AS ont été hybridées avec les fragments d'ADN amplifiés, digérées avec la RNase et les fragments ont été analysés sur du PAGE dénaturant. On a obtenu dans le cas d'une protection par l'exon 5 de hER en entier un fragment long de 140 nucléotides alors que dans le cas d'un mauvais appariemment au niveau du résidu amino-acide 400 on a obtenu 2 fragments (de 103 et 37 nucléotides). Dans ce dernier cas la digestion par la RNase n'a jamais été complète, résultant en la présence de bandes de 140 nucléotides, même dans les contrôles positifs. Les contrôles positifs ont été effectués en utilisant des échantillons d'ADN des plasmides HEGO et HEO (non montré sur le schéma, mais voir panneau B, colonnes 1 à 4).

(B) On a hybridé les échantillons de plasmides HEO (colonnes 1 et 3) et HEGO (colonnes 2 et 4) amplifiés par PCR, avec soit la sonde HEO AS (colonnes 1 et 2) ou la sonde HEGO AS (colonnes 3 et 4) et on les a digérés à la RNase. Des échantillons d'ADNc, amplifiés par PCR, de cellules HeLa (comme pour le contrôle négatif, colonnes 5 et 7) et de cellules MCF-7 (colonnes 6 et 8) ont été hybridés avec soit la sonde HEO AS (colonnes 5 et 6) ou la sonde HEGO AS (colonnes 7 et 8) et digérés à la RNase. Les échantillons d'ADN génomique PCR-amplifiés de cellules HeLa (colonnes 9 et 12), de cellules MCF-7 (colonnes 10 et 13) et de cellules LY2 (colonnes 11 et 14) ont été hybridés avec soit la sonde HEO AS (colonnes 9 à 11) ou la sonde HEGO AS (colonnes 12 à 14) et digérés avec une RNase. Les flèches noires indiquent le fragment protégé entier, long de 140 nucléotides, tandis que les pointes de flèches blanches pointent sur le fragment de 103 nucléotides qui s'est formé quand la RNase a coupé au niveau des mauvais appariemments dans les hybrides. M : marqueur de poids moléculaire.

## EXEMPLE 1 - MATERIEL ET METHODES VECTEURS D'EXPRESSION DES RECEPTEURS OESTROGENES ET PLASMIDES "REPORTER"

Les séquences d'ADNc codant pour les acides aminés de hER (Green et al., 1986) et de cER (Krust et al., 1986) sont subclonées dans le site EcoRI du vecteur d'expression eucaryote pSG1 (Green et al., 1988b) pour obtenir HEO et CEO, respectivement. On prépare de l'ADN simple brin et on pratique une mutagénèse dirigée comme cela a été décrit (Grundström et al., 1985) pour générer le vecteur d'expression HEGO de hER à partir de HEO, et le vecteur d'expression CEV de cER à partir de CEO en utilisant les oligonucléotides synthétiques :
5′-GGAGCACCCAG G GAAGCTACTGT-3′ et
5′-GGAACACCCAG TG AAGCTTTTATTT-3′
(les changements de nucléotides sont soulignés), respectivement.

Le séquences d'ADN sont vérifiées par séquençage en utilisant la technique aux didéoxydes. Vit-tk-CAT (Klein-Hitpass et al., 1986) et pYERE1/HER (Metzger et al., 1988) ont été décrits. On construit pYERE1/HEG en insérant les séquences d'ADNc hER de HEGO dans le vecteur parental décrit par Metzger et al. (1988).

## CULTURE DE CELLULE

On maintient les cellules HeLa et MCF-7 dans du milieu d'Eagle modifié de Dubelco (DMEM) supplémenté avec du sérum de veau foetal (FCS). 5 jours avant de les utiliser pour un dosage hormono-dépendant, on les transfère dans un milieu DMEM sans rouge de phénol, supplémenté avec du FCS traité au charbon enrobé de dextrane. LY2 est une cellule MCF-7 mutante sélectionnée par étapes pour sa résistance stable à l'anti-oestrogène LY117018; elle conserve ses récepteurs pour les oestrogènes (Bronzert et al., 1985). On maintient les cellules LY2 sur du milieu DMEM supplémenté avec du FCS traité au charbon enrobé de dextrane et 1 μM de tamoxifen. Les cellules de levure (souche TGY14.1) sont cultivées et transformées comme décrit (Ito et al., 1983; Metzger et al., 1988).

## DOSAGE DE LA FIXATION HORMONALE

On transfecte les cellules HeLa (boîtes de 9 cm) avec 10 μg de vecteurs d'expression du récepteur et 10 μg d'ADN transporteur ("Bluescribe M13 +", Stratagene) en utilisant la technique de précipitation au phosphate de calcium (Banerji et al., 1981). On prépare des extraits de cytosol à partir des cellules HeLa ou MCF-7 transfectées et on conduit le dosage de la fixation hormonale comme décrit (Green et al., 1986), excepté que le tampon d'homogénéisation contient également des inhibiteurs de protéase (2,5 μg/ml chacun, aprotinine, leuptine, chymostaine et antipaïne) et on incube des extraits aliquots (50 μl) à 4°C une nuit ou à 25°C pendant 2 heures avec des concentrations différentes de [³H]-17béta-oestradiol (148 Ci x mmol.⁻¹) avec ou sans un excès d'un facteur de 200 de 17béta-oestradiol non marqué.

## AMPLIFICATION PAR LE PCR

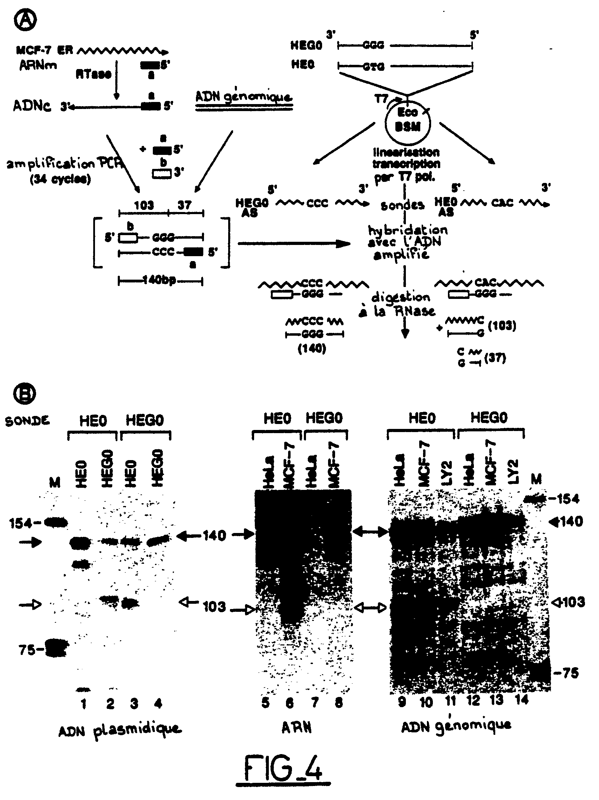

On isole l'ADN et l'ARN génomiques comme décrit par Miller et al., (1988) et Groudine et al., (1981), respectivement. On utilise 5 μg d'ARN pour préparer l'ADNc simple brin comme décrit par Huynh et al., (1985). La synthèse est amorcée avec l'oligonucléotide synthétique "a" de 21 bases (voir ci-dessous). En utilisant le PCR on amplifie les séquences correspondant à l'exon 5 du hER (Ponglikitmonkol et al., 1988), à partir de l'ADNc simple brin ou de 1 μg d'ADN génomique dans un volume réactionnel de 100 μl contenant : 10 mM de Tris-HCl pH 8,3, 50 mM de KCl, 1,5 mM de MgCl₂, 0,01 % (w/v) de gélatine, 200 μM de chaque dNTP, 1 μM de chaque amorce, 2,5 unités de Taq polymérase (Perkin Elmer/Cetus). Les amorces sont : "a" 5′-CTGTCCAAGAGCAAGTTAGGA-3′ et "b" 5′-GGCTTTGTGGATTTGACCCTC-3′ (voir aussi fig. 4A). Le PCR (Saiki et al., 1988) est pratiqué en utilisant un "Perkin Elmer/Cetus Thermal Cycler" et comprend une phase initiale de dénaturation pendant 90 secondes à 94°C, suivie par 34 cycles comprenant une dénaturation pendant 1 minutes à 94°C, une recuite pendant 2 minutes à 37°C et une élongation pendant 3 minutes à 72°C. On augmente à chaque cycle la période d'élongation de 4 secondes et la durée de la dernière période d'élongation est augmentée jusqu'à une durée de 9 minutes.

## CARTOGRAPHIE A L'AIDE DE LA RNase

L'ADNc de hER présent soit dans HEO ou HEGO est cloné dans le site EcoRI de "Bluescribe M13+" (Stratagene). On utilise les plasmides linéarisés avec XmnI dans le système de transcription in vitro à l'ARN polymérase T7 en suivant les inscriptions du fabricant de manière à générer uniformément des sondes d'ARN marquées au [32P]. On mélange I µl du produit du PCR avec environ 5 x 10⁵ cpm d'ARN marqué au [32P] dans 15 µl de solution d'hybridation (400 mM NaCl, 10 mM Pipes pH 6,5). On chauffe la solution à 85°C pendant 5 minutes et on la transfère immédiatement dans un bain d'eau maintenu à 68°C et incubé pendant 12 à 16 heures. La digestion à la RNase et l'analyse des fragments protégés sont effectuées comme décrit par Winter et al., (1985).

## AUTRES METHODES

Des transfections de cellules HeLa en utilisant la technique au phosphate de calcium, des dosages de CAT (Webster et al., 1988), l'analyse au Western blot (Green et al., 1988a) et le dosage de la béta-galactosidase (Metzger et al., 1988) sont pratiqués comme décrit.

## EXEMPLE 2

### STIMULATION APPAREMMENT OESTROGENE-INDEPENDANTE DE LA TRANSCRIPTION PAR LES RECEPTEURS AUX OESTROGENES POSSEDANT UN Gly-400 AU LIEU D'UN Val-400.

Pour tester si la présence de Val-400 au lieu de Gly-400 pouvait être responsable des besoins différents en oestradiol de HEO et CEO pour l'activation de la transcription, une mutagénèse dirigée a été utilisée pour remplacer le Val-400 de HEO par un résidu glycine (vecteur d'expression HEGO) et, réciproquement, pour remplacer le Gly-394 correspondant de CEO avec un résidu valine (vecteur d'expression CEV). A l'appui des hypothèses ci-dessus, HEGO s'est révélé activer la transcription approximativement de la même manière que CEO en absence d'oestrogène (comparer les colonnes 1, 7 et 13 dans la figure 1), tandis que CEV perdait la faculté du CEO d'activer la transcription en absence d'oestradiol et se comportait comme HEO (comparer les colonnes 1, 13 et 15).

Des antagonistes des oestrogènes ont été utilisés pour étudier si l'activation transcriptionnelle observée avec HEGO et CEO en l'absence d'ajout d'oestradiol pouvait être due à un niveau résiduel d'oestrogène restant dans le milieu de culture "dépouillé", duquel les hormones stéroïdes auraient dû être enlevées par traitement au charbon enrobé de dextrane. $10^{-8}$ M de 4-hydroxy-tamoxifen (OHT) (figure 1, comparer les colonnes 7 et 9) et $10^{-7}$ M de ICI 164,384 (ICI) (comparer les colonnes 7 et 11) ont tous deux supprimé l'activité apparemment oestrogène-indépendante de HEGO.

Il est à noter qu'en présence d'oestradiol à $10^{-8}$ M des ajouts similaires d'OHT à $10^{-8}$ M et d'ICI 164,384 à $10^{-7}$ M ont eu très peu d'effet sur l'induction à l'oestradiol (colonnes 10 et 12, respectivement).

## EXEMPLE 3

### LA PRESENCE DE Val-400 AU LIEU DE Gly-400 DIMINUE APPAREMMENT L'AFFINITE DE hER POUR OESTRADIOL A 25°C, MAIS PAS A 4°C

Les résultats ci-dessus nous ont suggéré que les récepteurs aux oestrogènes (ERs) avec Gly-400 (HEGO, CEO) peuvent montrer une plus grande affinité pour les oestrogènes que les ERs avec Val-400 (HEO, CEV), et par conséquent, pouvaient être activés dans une certaine mesure par des oestrogènes résiduels dans les milieux de culture "dépouillés". On a pratiqué à la fois des expériences in vivo d'activation transcriptionnelle et des études de fixation hormonale in vitro pour examiner cette possibilité. L'activation transcriptionnelle in vivo a été analysée à la fois dans des cellules HeLa en utilisant le gène "reporter" vit-tk-CAT et seulement 10 ng de vecteur d'expression d'ER (fig. 2A), et dans des levures en utilisant le système de dosage de la béta-galactosidase décrit par Metzger et al. (1988) (fig. 28). Dans les deux cas, on a obtenu une activation maximale de la transcription à une concentration d'oestradiol plus basse (E2) avec HEGO qu'avec HEO. Dans les cellules HeLa, une activité CAT maximale a été observée avec $10^{-10}$ M de E2 pour HEGO et avec de $10^{-9}$ à $10^{-8}$ M de E2 pour HEO (Fig. 2A, comparer colonnes 1-5 avec les colonnes 6-10). Pour les études de l'activation transcriptionnelle in vivo dans les levures, la séquence d'hER contenant Val-400 présente dans le plasmide pYERE1/HER (Metzger et al., 1988) fut remplacée par la séquence correspondante d'hER Gly-400 pour obtenir le plasmide pYERE1/HEG. On a obtenu la moitié de l'activité maximale de la béta-galactosidase avec environ 5nM de E2 pour la construction pYERE1/HEG Gly-400 et avec environ 40nM de E2 pour la construction pYERE1/HER Val-400 (Fig. 28 et données non communiquées). Il est particulièrement remarquable que, dans les deux cas, aucune activité de la béta-galactosidase n'a pu être détectée dans la levure en absence d'addition d'oestradiol, ce qui étaye la suggestion ci-dessus comme quoi l'activité transcriptionnelle observée dans les cellules HeLa avec HEGO en l'absence d'oestradiol était due à de bas niveaux d'oestrogène restant dans le milieu de culture "dépouillée".

L'affinité pour l'oestradiol des hERS contenant soit Val-400 (HEO) ou Gly-400 (HEGO) a été ensuite déterminée in vitro à la fois à 4°C et à 25°C, et comparée avec celle du hER endogène de cellule MCF-7 (fig. 3A, B et C). Des extraits de cytosol ont été préparés à partir de cellules HeLa transfectées avec soit HEO ou HEGO et à partir de cellules MCF-7 de cancer du sein. Les hERs d'HEGO et HEO exprimés dans des cellules HeLa (fig. 3A et B) ont toutes deux montré à 4°C la même affinité pour l'oestradiol que l'hER de MCF-7 ($K_D$ = 0,35 ± 0,05 nM, Fig. 3C) en accord avec les résultats déjà publiés comparant l'hER d'HEO et de cellules MCF-7 (Green et al., 1986). Cependant, une différence frappante dans l'affinité apparente pour l'oestradiol de HEGO et HEO a été observée à 25°C. Tandis que les affinités de hER d'HEGO et de MCF-7 pour l'oestradiol étaient similaires ( $K_D$ = 0,14 ± 0,02 nM ), la représentation de Scatchard de la fixation de l'oestradiol par HEO à 25°C n'a pas été une ligne droite, interdisant la détermination d'une valeur de $K_D$. D'une manière intéressante, à des concentrations saturantes d'oestrogène (de 5 à 10 nM) HEO fixait la même quantité de E2 à 25°C et à 4°C (fig. 3B), suggérant que la présence de l'hormone pourrait stabiliser HEO. De plus, à des concentrations saturantes d'oestradiol et quelle que soit la température d'incubation, l'extrait cytosolique HEGO fixait approximativement 5 fois plus d'oestradiol que l'extrait HEO correspondant (fig. 3A et B), même si les deux extraits contenaient approximativement les mêmes quantités de protéines hER comme on a pu en juger par les analyses au Western blot de ces extraits, effectuées soit avant ou après la période d'incubation à 25°C (fig. 3D).

Pour étudier plus avant si la présence d'oestradiol pouvait stabiliser la protéine HEO à 25°C, nous avons comparé la stabilité de protéines HEO et HEGO à 25°C en présence et en absence d'oestradiol. Des extraits cytosoliques de cellules HeLa transfectées avec soit HEO soit HEGO ont été tout d'abord incubées à 25°C sans ou avec 10nM d'oestradiol marqué (plus ou moins un extrait de E2 "froid") pendant 30 ou 60 minutes. De l'oestradiol marqué (10nM), plus ou moins un excès de E2 "froid", a été ensuite ajouté aux échantillons sans hormone, puis tous les échantillons ont été incubés pendant 2 heures de plus à 25°C, et la fixation spécifique de E2 a été déterminée (fig. 3E). HEGO a fixé l'oestradiol de la même manière, qu'il ait été ou non préincubé avec (triangles noirs) ou sans (triangles blancs) E2, tandis que HEO a fixé des quantités de E2 décroissantes quand il a été préincubé pendant 30 ou 60 minutes sans hormone (cercle blanc). Une telle décroissance dans la fixation de E2 n'a pas été observée quand E2 a été ajouté au temps 0 (cercle noir).

Ces résultats indiquent que l'hER d'HEO, mais pas le hER d'HEGO, a perdu irréversiblement ces capacités de fixation d'oestrogènes durant l'incubation à 25°C. Il est à noter que cette perte n'a pas été due à une protéolyse (voir ci-dessus, fig 3D)

## DISCUSSION

Les résultats des expériences de fixation hormonale in vitro étayent également la conclusion que hER contenant de la valine (codée par HEO) est différente de la majeure partie des hERs endogènes des cellules MCF-7. Bien que les valeurs du $K_D$ pour l'oestradiol mesurées à 4°C soient identiques pour HEO, HEGO (le hER contenant une glycine) et le hER endogène de cellules MCF-7, le $K_D$ pour HEO déterminé à 25°C est différent de celui du hER de HEGO et de cellules MCF-7.

La mutation créant une valine dans HEO affecte la capacité de fixation de l'oestradiol du récepteur à 25°C, mais pas à 0°C. Ceci n'est pas dû à une dégradation de la protéine durant la période d'incubation pour la fixation de l'oestradiol (fig. 3D), mais reflète probablement une instabilité de hER Val-400 à 25°C en l'absence d'oestradiol (fig. 3E), résultant en une perte de sa capacité à fixer l'oestradiol. L'incubation in vitro de HEO à 25°C sans oestradiol a réduit ses capacités de fixation de l'hormone ; toutefois, quand on a ajouté de l'oestradiol à des concentrations élevées, aucune perte de la fixation des oestrogènes n'a été observée (fig. 3E). Cette instabilité de hER HEO à 25°C explique très probablement l'observation comme quoi approximativement 5 fois moins d'oestradiol pouvait être fixé à la concentration optimale de ligand avec des extraits de cellules transfectées avec HEO en comparaison avec des cellules transfectées avec HEGO, même si les quantités de protéines HEO et

HEGO étaient identiques (fig. 3). Cette instabilité est probablement également responsable de la capacité réduite de hER codé par HEO synthétisé dans des levures à fixer l'oestradiol (Metzger et al., 1988).

Selon des prévisions relatives à la structure secondaire (Gibrat et al., 1987), le résidu aminoacide 400 de hER pourrait être situé dans un tour-béta entre une hélice alpha et une chaîne béta hydrophobe. Une recherche de ce motif dans une base de données structurelles et la construction subséquente du modèle de la séquence d'aminoacides d'hER autour de la position 400 suggère que Pro-399 et Gly-400 occupent les deux positions centrales d'un tour-béta de type II comme on en trouve souvent dans les structures protéiques (Richardson, 1981). Ainsi, la présence d'un résidu valine en position 400 pourrait déstabiliser cette partie de la structure de l'ER qui pourrait résulter en une dénaturation thermique à 25 °C et donc en une perte de la possibilité de fixation de l'hormone. On ignore si l'ensemble hélice-alpha-tour-béta-chaîne-béta de hER qui contient le résidu Gly-400 pourrait être directement impliqué dans la fixation de l'oestradiol. Le fait que la fixation de l'hormone stabilise hER mutant Val-400 suggère, toutefois, que la structure tridimensionnelle de cette région de hER est influencée par la fixation du ligand.

Les présents résultats offrent une explication satisfaisante à l'observation que HEGO (hER de type sauvage), de même que le récepteur aux oestrogènes du poulet, mais pas le mutant HEO d'hER, sont capables d'activer la transcription du gène "reporter" vit-tk-CAT quand les cellules transfectées HeLa sont maintenues dans le milieu de culture dépourvu d'hormone stéroïde (fig. 1). Dû à son instabilité en l'absence d'oestradiol, HEO ne peut pas fixer effectivement les oestrogènes qui sont présents en concentration très limitante dans le milieu de culture "dépouillé", au contraire de HEGO qui est stable en l'absence d'oestradiol et qui, par conséquent, peut activer la transcription même sous des conditions limitantes en oestrogène. Le fait que des oestrogènes résiduels soient en réalité présents dans le milieu de culture "dépouillé" est indiqué à la fois par les effets inhibiteurs des antagonistes hydroxy-tamoxifen et ICI 164,348 sur l'activité apparemment constitutive de HEGO dans les cellules HeLa (fig. 1), et par l'inactivité complète du même récepteur dans les cellules de levures qui sont cultivées dans un milieu totalement dépourvu d'oestrogène (fig. 2B).

Finalement, il est important de remarquer qu'une comparaison des propriétés de fixation à l'ADN et d'activation transcriptionnelle de l'hER Gly-400 de type sauvage (vecteur d'expression HEGO) avec celle précédemment observée pour l'hER Val-400 [vecteur d'expression HEO (Green et Chambon, 1987; Kumar et al., 1987; Kumar et Chambon, 1988; Tora et al., 1988; Webster et al., 1988; Mader et al., 1989; Webster et al., 1989)] n'a pas révélé, jusqu'à présent, de différences significatives.

## REFERENCES

Banerji, J., Rusconi, S. and Schaffner, W. (1981) Cell 27 , 299-308.

Bronzert, D.A., Greene, G.L. and Lippman, M.E. (1985) Endocrinology 117 , 1409-1417.

Evans, R.M. (1988) Science 240 , 889-895.

Gibrat, J.F., Garnier, J. and Robson, B. (1987). J. Mol. Biol. 198 , 425-443.

Green, S., Walter, P., Kumar, V., Krust, A., Bornert, J.M., Argos, P. and Chambon, P. (1986) Nature 320 , 134-139.

Green, S. and Chambon, P. (1987) Nature 325 , 75-78.

Green, S. and Chambon, P. (1988) Trends in Genetics 4 , 309-314.

Green, S., Kumar, V., Theulaz, I., Wahli, W. and Chambon, P. (1988a) EMBO J. 7 , 3037-3044.

Green, S. Issemann, I. and Sheer E. (1988b) Nucl. Acids Res. 16 , 369.

Greene, G.L., Sobel, N.B., King, W.J. and Jensen, E.V. (1984) J. Steroid Biochem. 20 , 51-56.

Greene G.L., Gilna, P, Waterfield, M., Baker, A., Hort, Y. and Shine, J. (1986) Science 231 , 1150-1154.

Gronemeyer, H., Green, S., Jeltsch, J.M., Kumar, V., Krust, A. and Chambon, P. (1988) In Gronemeyer, H. (ed.), Affinity labeling and cloning of steroid and thyroid hormone receptors : techniques, application and functional analysis. Ellis Horwood, pp. 252-297.

Groudine, M., Peretz, M. and Weintraub, M. (1981) Mol. Cell. Biol. 1 , 281-288.

Grundström, T., Zenke, W.M., Wintzerith, M., Matthes, H.W.D., Staub, A. and Chambon, P. (1985) Nucl. Acids Res. 13 , 3305-3316.

Huynh, T.V., Young, R.A. and Davis, R.W. (1985) In Glover, D.M. (ed.), DNA cloning : a practical approach. IRL Press Oxford, pp. 49-78.

Ito, M., Fukuda, Y., Murata, K. and Kimura, A. (1983) J. Bacteriol. 153 , 163-168.

Klein-Hitpass, L., Schorpp, M., Wagner, U. and Gerhart, U.R. (1986) Cell 46 , 1053-1061.

Koike, S., Sakai, M. and Muramatsu, M. (1987) Nucl. Acids Res. 15 , 2499-2513.

Kumar, V., Green, S., Staub, A. and Chambon, P. (1986) EMBO J. 5 , 2231-2236.

Kumar, V., Green, S., Stack, G., Berry, M., Jin, J.R. and Chambon, P. (1987) Cell 51 , 941-951.

Kumar, V. and Chambon, P. (1988) Cell 55 , 145-156.

Krust, A., Green, S., Argos, P., Kumar, V., Walter, P., Bornert, J.M. and Chambon, P. (1986) EM80 J. 5 , 891-897.

Mader, S., Kumar, V., de Verneuil, H. and Chambon, P. (1989) Nature, in press.

Metzger, D., White, J.H. and Chambon, P. (1988) Nature 334 , 31-36.

Miller, S.A., Dykes, D.D. and Polesky, H.F. (1988) Nucl. Acids Res. 16 , 1215.

Petkovich, M., Brand, N.J ., Krust, A. and Chambon, P. (1987) Nature 330 , 444-450.

Ponglikitmongkol, M., Green S. and Chambon, P. (1988) EMBO J. 7 , 3385-3388.

Richardson, J.S. (1981) Adv. in Prot. Chemistry 34 , 167-339.

Roberts, J.D., Bebenek, K. and Kunkel, T.A. (1988) Science 242 , 1171-173.

Saiki, R.K., Gelfand, D.H., Stoffel, S., Scharf, S.J., Higuchi, R., Horn, G.T., Mullis, K.B. and Erlich, M.A. (1988) Science 239 , 487-491.

Tora, L., Gaub, M.P., Mader, S., Dierich, A., Bellard, M. and Chambon, P. (1988) EMBO J. 7 , 3771-3778.

Walter, P., Green, S., Greene, G., Krust, A., Bornert, J.M., Jeltsch, J.M., Staub, A., Jensen, E., Scrace, G., Waterfield, M. and Chambon, P. (1985) Proc. Natl. Acad. Sci. USA 82 , 7889-7893.

Webster, N.J.G., Green, S., Jin, J.R. and Chambon, P. (1988) Cell 54 , 199-207.

Webster, N.J.G., Green, S., Tasset, D., Ponglikitmongkol, M. and Chambon P. (1989) EMBO J., in press.

Weiler, I.J., Lew, D. and Shapiro, D.J. (1987) Mol. Endo. 1 , 355-362.

White, R., Lees, J.A., Needham, M., Ham, J. and Parker, M. (1987) Mol. Endo. 1 , 735-744.

Winter, E., Yamamoto, F., Almoguera, C. an Perucho, M. (1985) Proc. Natl. Acad. Sci. USA 82 , 7575-7579.

## Revendications

1. Procédé d'obtention du récepteur humain natif sauvage des oestrogènes, caractérisé en ce qu'on cultive des cellules recombinantes incorporant l'ADN codant pour les récepteurs humains natifs sauvages des oestrogènes comportant une glycine en position 400 sous le contrôle d'élément assurant l'expression de cet ADN dans les cellules en cause, et en ce que l'on purifie ledit récepteur si nécessaire.

2. Procédé selon la revendication 1, caractérisé en ce que les cellules recombinantes sont des cellules animales, des cellules de levures ou d'autres microorganismes.

3. Procédé selon la revendication 2, caractérisé en ce que les cellules sont des cellules HeLa.

4. Procédé selon l'une des revendication 1 à 3, caractérisé en ce que la glycine en position 400 est codé par la séquence GGG.

5. Cellule recombinante contenant le récepteur humain natif sauvage des oestrogènes ou le domaine isolé de liaisons des oestrogènes obtenus dans le procédé selon l'une des revendications 1 à 4.

6. Récepteur humain natif sauvage des oestrogènes obtenus par le procédé selon l'une des revendications 1 à 5.

7. Application des cellules selon la revendication 5 ou des récepteurs selon la revendication 6, à titre de modèle pour tester les agonistes et les antagonistes des oestrogènes.

8. Application des cellules ou des récepteurs selon la revendication 7, caractérisée en ce que le modèle est utilisé dans des conditions physiologiques.

9. Application des cellules selon la revendication 5 ou d'un récepteur selon la revendication 6 pour standardiser les immunoessais utilisés dans le diagnostic et le pronostic des tumeurs hormonaux dépendantes.

FIG_1

CELLULES HELA

FIG_2A

FIG_2B

FIG_3

FIG_4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| P,X | THE EMBO JOURNAL vol. 8, no. 7, 1989, pages 1981-1986, Oxford, GB; L. TORA et al.: "The cloned human oestrogen receptor contains a mutation which alters its hormone binding properties" * le document en entier * | 1-9 | C 12 N  15/12 C 12 P  21/02 |
| P,X | EP-A-0 349 435  (CENTRE NATIONAL DE LA RECHERCHE SCIENTIFIQUE) * le document en entier, en particulier page 4, lignes 15-28; page 10, exemple 5 * | 1-9 | |
| D,Y | WO-A-8 705 049  (CALIFORNIA BIOTECHNOLOGY INC.) * le document en entier * | 1-9 | |
| D,Y | THE EMBO JOURNAL vol. 7, no. 11, 1988, pages 3385-3388, Oxford, GB; M. PONGLIKITMONGKOL et al.: "Genomic organization of the human oestrogen receptor gene" * abrégé; page 3386, colonne 2 - page 3387, colonne 1 * | 1-9 | **DOMAINES TECHNIQUES RECHERCHES (Int. Cl.5)** C 12 N  15/12 C 12 P  21/00 C 12 P  21/02 |
| D,A | THE EMBO JOURNAL vol. 5, no. 5, 1986, pages 891-897, Oxford, GB; A. KRUST et al.: "The chicken oestrogen receptor sequence: homology with v-erbA and the human oestrogen and glucocorticoid receptors" * page 892, colonne 2; figure 2; page 895, colonne 2 * | 1,6 | |

-/-

Le présent rapport a été établi pour toutes les revendications

| Lieu de la recherche | Date d'achèvement de la recherche | Examinateur |
|---|---|---|
| BERLIN | 07-09-1990 | JULIA P. |

Office européen
des brevets

**RAPPORT DE RECHERCHE EUROPEENNE**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | CHEMICAL ABSTRACTS vol. 110, no. 13, 27 mars 1989, page 494, abrégé no. 112527y, Columbus, Ohio, US; R. PIVA et al.: "Abnormal methylation of estrogen receptor gene and reduced estrogen receptor RNA levels in human endometrial carcinomas" & J. Steroid Biochem. 1989, vol. 32, no. 1A, pages 1-4 --- | 1 | |
| A | PASCAL DATABASE abrégé no. 88-X-0220459; F.F. PARL et al.: "Detection of estrogen receptor mRNA in human uterus" & Mol. Cell. Endocrinol. 1987, vol. 52, no. 3, pages 235-242 ----- | 1 | |
| | | | **DOMAINES TECHNIQUES RECHERCHES (Int. Cl.5)** |

Le présent rapport a été établi pour toutes les revendications

| Lieu de la recherche | Date d'achèvement de la recherche | Examinateur |
|---|---|---|
| BERLIN | 07-09-1990 | JULIA P. |